# EUROPEAN PATENT APPLICATION

(11) **EP 1 757 913 A1**
(43) Date of publication of application: **28.02.2007**
(21) Application number: 05748457.8
(22) Date of filing: 06.06.2005
(51) Int. Cl.: G01J 3/10, A61C 19/04, G03B 15/02

(54) **LIGHTING UNIT, AND IMAGE PICKUP DEVICE**

(30) Priority: 15.06.2004 JP 2004176930; 12.07.2004 JP 2004204290
(71) Applicant: Olympus Corporation, Tokyo 151-0072 (JP)
(72) Inventor: OKAMURA, Toshiro, 1910043 (JP); WADA, Toru, 3520004 (JP)
(74) Representative: von Hellfeld, Axel
(86) International application number: PCT/JP2005/010736
(87) International publication number: WO 2005/124299

(57) **Abstract**

There is provided a small sized imaging apparatus which can measure with high accuracy a color distribution of a surface of an object, in which a light intensity distribution on a predetermined surface in a direction substantially perpendicular to an optical axis is uniform, and a change in an amount of light in a direction along the optical axis is reduced, and an illuminating unit which used in this imaging apparatus. (The imaging apparatus) Includes a light source section (210) which supplies illuminating light, a diffusing section (211) which diffuses by reflecting the illuminating light from the light source section (210), and aperture sections (212a and 212b) which allow to emerge diffused illuminating light, and the aperture sections (212a and 212b) has an aperture diameter D which allows the diffused illuminating light to emerge as a substantially parallel light.

## Description

### TECHNICAL FIELD

The present invention relates to an illuminating unit, and an imaging apparatus which includes the illuminating unit.

### BACKGROUND ART

For example, paint on a surface of a passenger automobile, a gloss and a color of a tooth of a human being has been hitherto measured over a predetermined range. Moreover, based on measurement results, an unevenness of the gloss, an unevenness of the color of the tooth, and an unevenness of the paint of an object is calculated. Particularly, in a field of dental treatment, many a times, a dentist makes a judgment of the color of tooth, a color tone, and the gloss. For this, a predetermined range of tooth is illuminated by an illuminating unit. A light emitting diode (hereinafter called as "LED") can be used as a light source of the illuminating unit. A structure of a color measuring apparatus in which the LED is used is proposed in Japanese Patent No. 3218601.

The unevenness of the color, the color tone, and the gloss is calculated based on a light scattered from the surface. Optical characteristics of the surface of the object, for example "a distribution of the color, the color tone, and the gloss" will be hereinafter called generically as "color distribution". Therefore, for measuring accurately the color distribution of the surface of the object, it is necessary to illuminate by light of a uniform intensity distribution.

However, normally, spatial light distribution characteristics of the light from the LED are not uniform. Therefore, the intensity distribution of the light from the LED has non-uniformity to certain extent. In the structure of the Japanese Patent No. 3218601 mentioned above, light of three primary colors from the LED is irradiated directly on a surface to be irradiated of the object. Therefore, an unevenness of the light intensity distribution is developed on the surface to be irradiated of the object. As a result of this, the color distribution of the surface of the object cannot be measured accurately.

Moreover, for example, in many cases the dentist measures the color distribution of a tooth by holding an imaging apparatus in a hand. At this time, the imaging apparatus is not fixed mechanically. As a result of this, a gap between the imaging apparatus and the tooth which is an object, in other words an imaging distance along a direction of an optical axis, is changed sometimes during the measurement of the color distribution. Moreover, in the structure in Japanese Patent No. 3218601, when a gap between a color measuring apparatus and the object is changed, the unevenness in the light intensity distribution of the illuminated light is increased. Thus, the structure in the Japanese Patent No. 3218601 is a problem due to an occurrence of the uneven light intensity distribution caused by a change in the imaging distance along the direction of the optical axis. This problem becomes even more remarkable when the object is three-dimensional.

Furthermore, when the illuminating unit illuminates the surface of the object, a scattered light and a specularly(regularly) reflected light are generated. The unevenness of the paint of the object, or the unevenness of the color, the color tone, and the gloss are calculated based on the scattered light from the surface. Therefore, at the time of measuring the color distribution of the surface of the object, it becomes necessary to capture an image of the scattered light from the surface. Whereas, the specularly reflected light from the surface of the object has the light intensity substantially higher as compared to the scattered light. As a result of this, when the specularly reflected light (which is reflected) from the surface is incident on an imaging optical system, the color distribution of the surface cannot be measured accurately.

In the structure in the Japanese Patent No. 3218601, an angle between an optical axis of the LED which is an illuminating optical system, and a central axis of a photodiode of an imaging element is small. Therefore, out of the light irradiated from the LED, the light which is specularly reflected at the surface of the object is incident on the photodiode. As a result of this, the unevenness of the color of the surface of the object cannot be measured accurately, which is a problem. Particularly, when the object has a gloss, the light intensity of the specularly reflected light becomes higher. Therefore, in the structure of the conventional technology, in a case of the object having a gloss, this problem becomes even more remarkable.

For reducing the specularly reflected light from the surface of the object, widening an angle between the optical axis of the illuminating optical system, and an optical axis of an imaging optical system can be taken into consideration. Here, sometimes the object is a three-dimensional structure. In this case, when the angle between the optical axis of the illuminating optical system and the optical axis of the imaging optical system is wide, a shadow portion occurs in the object. The shadow portion of the object cannot acquire information from the surface.

Thus, it is necessary that the shadow is not developed in the object, while reducing the specularly reflected light. Therefore, disposing the illuminating optical system in two directions facing each other, with the imaging optical system as a center can be taken into consideration. The illuminating light from the illuminating optical system is let to be incident obliquely on the object. Accordingly, it is possible to reduce the specularly reflected light, and to avoid the shadow from developing on the object. However, it is necessary to dispose at least two illuminating optical systems around the imaging optical system. As a result of this, there is a problem that a size of the imaging apparatus becomes big. Particularly, in the field of dental treatment where a tooth is let to be the object, a reduction in the size of the imaging apparatus is desired strongly.

The present invention is made in view of the abovementioned problems, and it is an object of the present invention to provide an imaging apparatus of a small size which is capable of measuring with high accuracy a color distribution of a surface of an object, in which a light intensity distribution on a predetermined surface in a direction substantially perpendicular to an optical axis is uniform, and a change in an amount of light in a direction along the optical axis is reduced, and an illuminating unit which is used in this imaging apparatus.

Moreover, another object of the present invention is to provide an imaging apparatus of a small size which is capable of measuring with high accuracy a color distribution of a surface of an object by a bright diffused light, and an illuminating unit which is used in this imaging apparatus.

### DISCLOSURE OF THE INVENTION

For solving the abovementioned issues, and attaining the object, according to a first invention, it is possible to provide an illuminating unit which includes a light source section which supplies an illuminating light, a diffusing section which diffuses by reflecting the illuminating light from the light source section, and an aperture section which allows the diffused illuminating light to emerge, and the aperture section has a diameter which allows the diffused illuminating light to emerge as a substantially parallel light.

Moreover, according to a second invention, it is possible to provide an illuminating unit which includes a light source section which supplies an illuminating light, a diffusing section which diffuses by reflecting the illuminating light from the light source section, and an aperture section which allows the diffused illuminating light to emerge, and the light source section is provided at a position at which the illuminating light supplied by the light source section is emerged from the aperture section upon being reflected at least once in the diffusing section.

Furthermore, according to a third invention, it is possible to provide an imaging apparatus which includes the illuminating unit, and an imaging optical system which forms an image of a reflected light from an irradiated surface which is illuminated by the illuminating unit, and a color distribution of the irradiated surface is calculated based on the reflected light acquired by the imaging optical system.

There is shown an effect that the imaging apparatus according to the present invention can measure with high accuracy a color distribution of a surface of an object, in which a light intensity distribution on a predetermined surface in a direction substantially perpendicular to an optical axis is uniform, and a change in amount of light in a direction along the optical axis is reduced. Moreover, there is shown an effect that the imaging apparatus according to the present invention is small sized, and can measure with accuracy a color distribution of a surface of an object by a bright diffused light.

### BRIEF DESCRIPTION OF THE DRAWINGS

Fig. 1 is a diagram showing a formation of a system which uses an imaging apparatus according to a first embodiment;
Fig. 2 is a block diagram of the system which uses the imaging apparatus according to the first embodiment;
Fig. 3 is a diagram showing briefly an optical system portion of the first embodiment;
Fig. 4 is a perspective structure diagram of an illuminating unit of the first embodiment;
Fig. 5 is a top surface view of the illuminating unit of the first embodiment;
Fig. 6 is a cross-sectional structure diagram of the illuminating unit of the first embodiment;
Fig. 7A is a diagram showing a relation between a size of an aperture section and an emerged light;
Fig. 7B is another diagram showing the relation between the size of the aperture section and the emerged light;
Fig. 8 is a diagram showing a spectral distribution of an LED;
Fig. 9 is a diagram showing a relation between a position of the aperture section and an intensity distribution at an optical axis;
Fig. 10A is a diagram showing a cross-sectional structure of a diffusing section of the first embodiment;
Fig. 10B is a diagram showing a cross-sectional structure of another diffusing section of the first embodiment;
Fig. 10C is a diagram showing a cross-sectional structure of still another diffusing section of the first embodiment;
Fig. 11 is a top surface view of an illuminating unit of a second embodiment;
Fig. 12 is a cross-sectional structure diagram of the illuminating unit of the second embodiment;
Fig. 13 is a cross-sectional structure diagram of an illuminating unit of a third embodiment;
Fig. 14A is a diagram showing a structure of an LED of the third embodiment;
Fig. 14B is a diagram showing another structure of the LED of the third embodiment; and
Fig. 14C is a diagram showing still another structure of the LED of the third embodiment.

### BEST MODE FOR CARRYING OUT THE INVENTION

Embodiments of an illuminating unit and an imaging apparatus according to the present invention will be described below in detail with reference to the accompanying diagrams. However, the present invention is not restricted to the embodiments described below.

### (First embodiment)

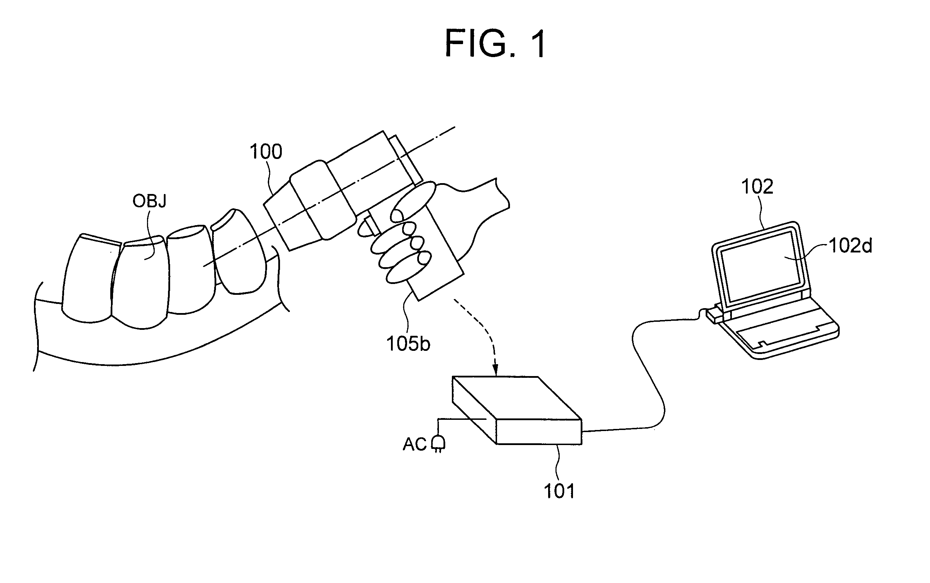

Fig. 1 shows a formation for use of an imaging apparatus 100. The imaging apparatus 100 is an apparatus used for application of measuring accurately a gloss and a color of a tooth of a human being as an object OBJ, for example.

As shown in Fig. 1, a system which uses the imaging apparatus 100 includes the imaging apparatus 100, a cradle 101, and a computer 102. The imaging apparatus 100 performs imaging of the object OBJ such as a tooth. After the imaging, the imaging apparatus 100 is mounted on the cradle 102. Accordingly, the cradle 101 is electrically connected to the imaging apparatus 100. By electrically connecting the cradle 101 to the imaging apparatus 100, the cradle 102 receives imaging data from the imaging apparatus 100. At the same time, the cradle 101 charges the imaging apparatus 100.

The computer 102 is connected to the cradle 101. The computer 102 receives the imaging data via the cradle 101. Next, the computer 102 performs a predetermined analysis process based on the imaging data.

By using such a system, for example, a dentist performs imaging of a surface of a human tooth and performs the analysis process. Here, a dental treatment which recuperates the damaged tooth is considered. At this time, it is desirable that the tooth before the recuperation and the tooth after the recuperation, such as an artificial tooth, have the substantially same color distribution. So far, the artificial tooth has been manufactured based on a subjective judgment by the dentist. At the time of making the judgment, sometimes an artificial tooth in which a color distribution with an adjacent tooth is not matched due to a difference in an illuminating environment when an original tooth is observed and when the artificial tooth is manufactured, and a difference of the observing person, is manufactured, and the artificial tooth is to be remade.

Whereas, the dentist who uses this system, measures the color distribution of the original tooth before recuperation, or measures the color distribution of the tooth which is adjacent to the tooth to be recuperated. Further, an artificial tooth having the color distribution matching with the color distribution acquired by the analysis process is manufactured. Thus, according to this system, it is possible to compare the color distribution of the tooth after the recuperation and the color distribution of the tooth before the recuperation based on objective image data, without depending on the subjective judgment of the dentist. As a result, by using this system, it is possible to prevent the mismatching of the color of the artificial tooth due to the difference of a person and the difference of the illuminating environment, and to reduce a possibility of remaking the artificial tooth.

Moreover, in recent years, in dentistry, a so-called whitening process which is a treatment of processing a surface of a tooth to a natural white color has been performed. Once a patient is used to the color of the tooth after the treatment, the patient feels that the color has been the original color of the tooth, and is sometimes skeptical about an effect of the treatment. At the time of performing the whitening process, the dentist by using this system can check objectively the whiteness of the tooth after the process, and some times as a numerical value. Consequently, the patient who has received the whitening treatment can realize the effect as an objective numerical value, and it is possible to reduce an unwanted doubt and dissatisfaction due to a vague memory and a wrong impression.

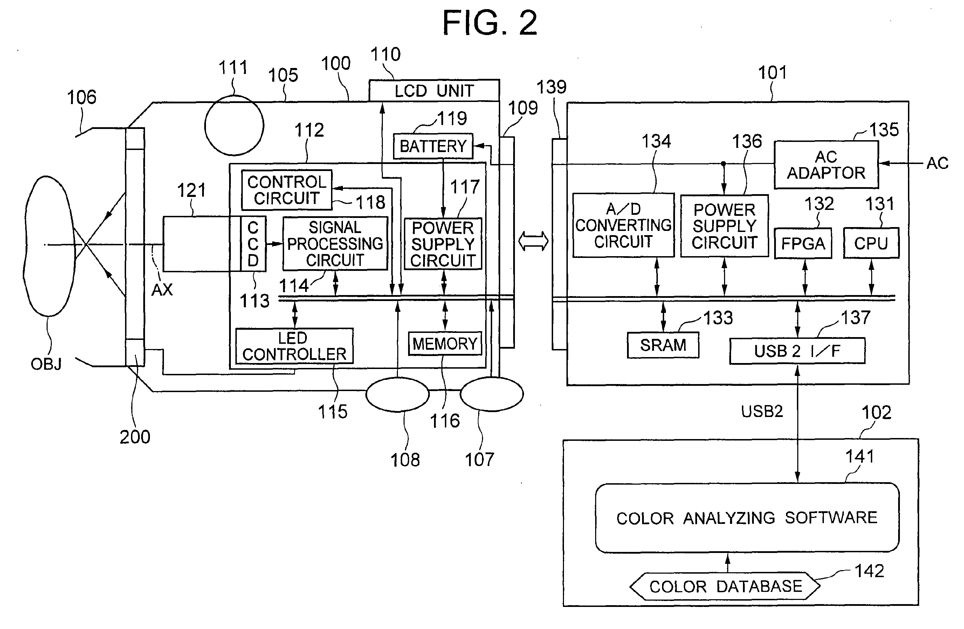

Next, a system which uses the imaging apparatus 100 will be described by referring to Fig. 2. Fig. 2 is a block diagram showing a schematic structure of this system.

A hood 106 for the object OBJ is installed in an extended form on a main body 105 of the imaging apparatus 100. The obj ect OBJ is, for example, a tooth in the dental treatment. Moreover, a power supply switch 107, a shutter button 108, a contact point 109, an LCD unit 110, and a focus ring 111 are formed on an outer surface of the main body 105. The power supply switch 107 puts ON or OFF a power supply of the imaging apparatus 100. The shutter button 108 makes an indication input of an imaging operation. The contact point 109 electrically connects the cradle 101 and the imaging apparatus 100. The LCD unit 110 performs a display of an image which is captured, and a display of various types of information related to the imaging apparatus 100. Moreover, by an operation of the focus ring 111, it is possible to adjust manually a focal position of an imaging optical system 121 which will be described later.

An illuminating unit 200, the imaging optical system 121, and a CCD 113 are disposed inside the main body 105. The illuminating unit 200 irradiates illuminating light which illuminates the object OBJ. A detailed structure of the illuminating unit 200 will be described later. The imaging optical system 121 forms on a predetermined surface an image of a scattered light from a surface of the object OBJ which is illuminated. The imaging optical system 121 has a lens structure which can form an image of the object OBJ at the most close distance. The CCD 113 which is an imaging device performs imaging of the object OBJ by light which is transmitted through the imaging optical system 121. Here, the predetermined surface or a surrounding of the predetermined surface and a light receiving surface of the CCD 113 are disposed to coincide. The CCD 113 converts an image of the object OBJ which is formed by imaging optical system 121, to an electrical image signal.

The hood 106 shields unnecessary external light. Accordingly, the hood 106 can guide efficiently only the scattered light which is reflected from the surface of the object OBJ to the imaging optical system 121. Moreover, it is possible to adjust a position of the imaging optical system by operating manually the focus ring 111 by an operator such as a dentist. An imaging position of the object OBJ by the imaging optical system 121, and the light receiving surface of the CCD 113 are allowed to coincide by the operation of the focus ring 111. Instead of using the focus ring 111, a structure which can adjust a focal point automatically by a heretofore known auto-focusing mechanism may be used.

Furthermore, an electric circuit board 112 is provided inside the main body 105. A signal processing circuit 114, an LED controller 115, a controlling circuit 118, a memory 116, and a power supply circuit 117 are installed on the electric circuit board 112.

The signal processing circuit 114 performs various signal-processing of an image signal which is output from the CCD 113. The LED controller 115 controls a light emission state of an LED included in the illuminating unit 200. The memory 116 performs storing of image data processed by the signal processing circuit 114, and storing of data and processing computer program executed by a control circuit 118 which will be described later. A battery 119 accumulates a power supply which is to be supplied from the cradle 101 to the imaging apparatus 100 via the contact point 109. The power supply circuit 117 supplies the power supply which is supplied from the battery 119 to each circuit inside the imaging apparatus 100. Moreover, the control circuit 118 is connected in both directions to the illuminating unit 100, the signal processing circuit 114, the LED controller 115, the memory 116, and the power supply circuit 117, via a bus etc. The control circuit 118 performs a comprehensive control of the entire imaging apparatus 100.

As shown in Fig. 1, a grip portion 105b for the operator to hold in a hand is provided on the imaging apparatus 100. The shutter button 108 is disposed at a position operable by a forefinger etc. of the hand holding the grip portion 105b. Moreover, the power supply switch 107 is disposed at a position on a side opposite to the shutter button 108, such as a position operable by a thumb etc. of the hand holding the grip portion 105b for example. Furthermore, the contact point 109 is provided on the grip portion 105b. The battery 119 is disposed inside the grip portion 105b. Moreover, the LCD unit 110 is disposed at an easily observable position on a rear side of the main body 105 of the imaging apparatus 100.

Next, the cradle 101 will be described. A contact point 139, an AC adaptor 135, an A/D converting circuit 134, and a power supply circuit 136 are disposed in the cradle 101. The contact point 139 is electrically connected to the contact point 109 of the imaging apparatus 100. The AC adaptor 135 converts a predetermined AC voltage which is supplied from an AC power supply to an appropriate DC voltage. The A/D converting circuit 135 performs a conversion to digital data when the image data transmitted from the imaging apparatus 100 is analog data. The power supply circuit 136 supplies the power supply supplied from the AC adaptor 135, to each circuit therein.

An SRAM 133, an FPGA (Field Programmable Gate Array) 132, an interface (I/F) 137 of a USB 2, and a CPU 131 are further disposed in the cradle 101. The SRAM 133 performs storing of image data and storing of data and processing computer program executed by the CPU 131 which will be described later. The FPGA 132 performs a compression process of the image data. The I/F 137 of the USB 2 performs communication with the computer 102 by the USB 2 for example. Moreover, the CPU 131 is connected in both directions to the FPGA 132, the SRAM 133, the A/D converting circuit 134, the power supply circuit 136, and the I/F 137, via a bus etc. The CPU 131 performs a comprehensive control of the entire cradle 102 and a control of communication with the imaging apparatus 100, and a control of communication with the computer 102.

Moreover, the computer 102 is connected to the cradle 101 via the USB 2 for example. A color analyzing software 141 is installed in the computer 102. The color analyzing software 141 performs an analysis process of the image data received from the imaging apparatus 100. Accordingly, it is possible to calculate a color distribution of the object OBJ. Moreover, a color database 142 is stored in a memory of the computer 102 which is not shown in the diagram. The color analyzing software 141 refers to the color database 142 at the time of performing the analysis process of the image data.

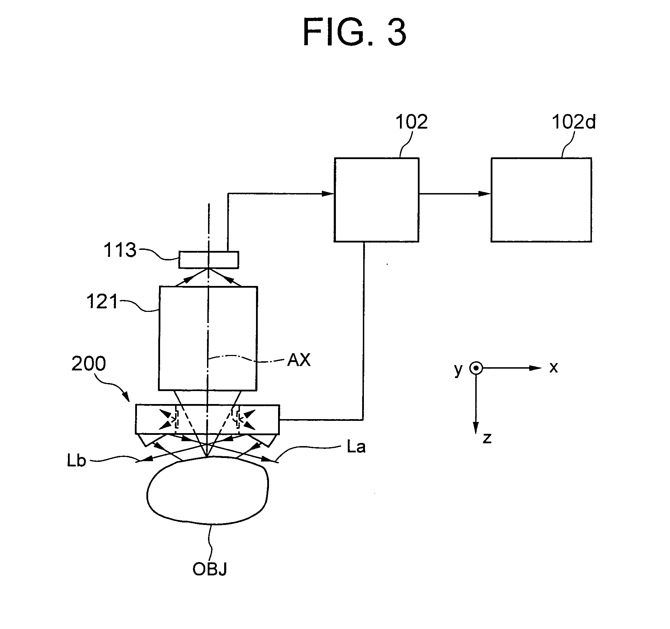

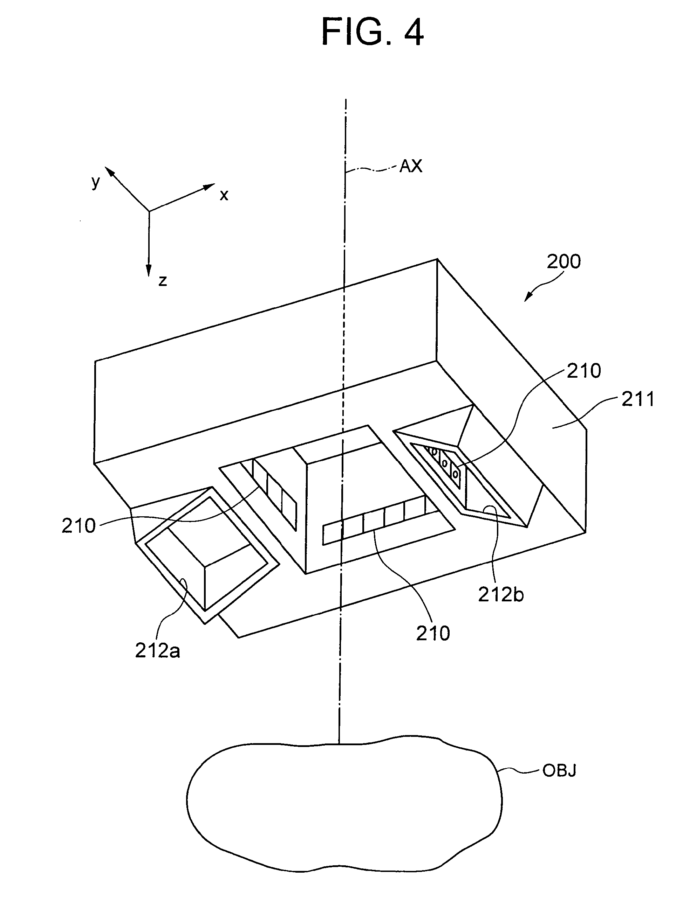

Fig. 3 shows briefly a structure mainly of an optical system, in the system which uses the imaging apparatus according to the first embodiment. The illuminating unit 200 illuminates the object OBJ. Details of the illuminating unit 200 will be described by using Fig. 4, Fig. 5, and Fig. 6. Scattered light from the object OBJ passes through a central portion of the illuminating unit 200. The scattered light which is passed is incident on the imaging optical system 121. Light emerged from the imaging optical system 121 is imaged at the CCD 113. Moreover, the illuminating unit 200 is provided in an optical path between the CCD 113 which is an imaging optical system, and an irradiated surface of the object OBJ.

As it will be described later, the illuminating unit 200 sequentially illuminates the object OBJ by light in a predetermined wavelength range. The scattered light reflected from the object OBJ which is illuminated by the light in the predetermined wavelength range is incident on the imaging optical system 121. Next, light from the imaging optical system 121 is received at the CCD 113. Further, as it has been described above, the computer 102 performs an analysis process of the color distribution of the surface of the object OBJ based on the image data from the CCD 113. The color distribution of the tooth which is an analysis result is displayed on a display 102d.

### (Structure of illuminating unit)

Fig. 4 shows a perspective structure of the illuminating unit 200 as seen from a side of the object OBJ. The illuminating unit 200 has a light source section 210, a diffusing section 211, a first aperture section 212a, and a second aperture section 212b. The light source section 210 is made of a plurality of LEDs which supply the illuminating light. The diffusing section 211 diffuses by reflecting the illuminated light from the light source section 210. The first aperture section 212a and 212b allows the diffused illuminating light to emerge. The diffusing section 211 is structured to be square cylindrical shaped. Moreover, a center of a hollow portion of the diffusing section 211 and an optical axis AX of an imaging optical system which is not shown in the diagram are allowed to coincide substantially.

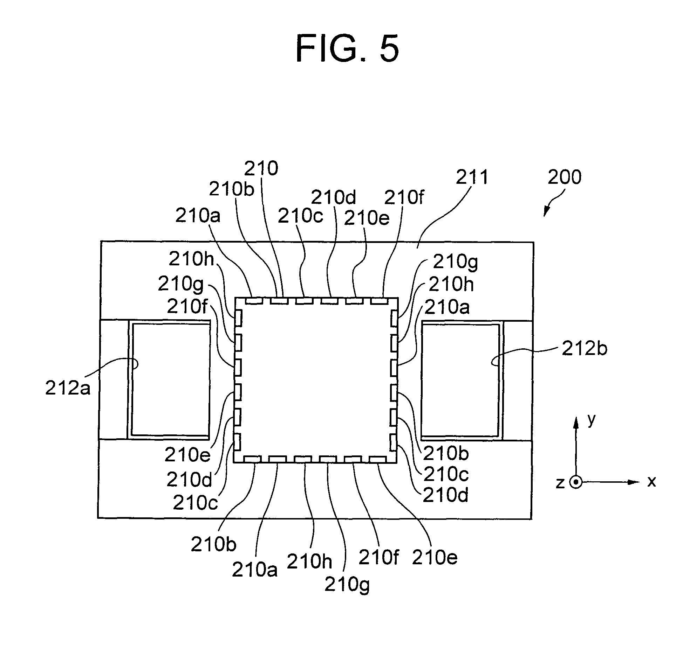

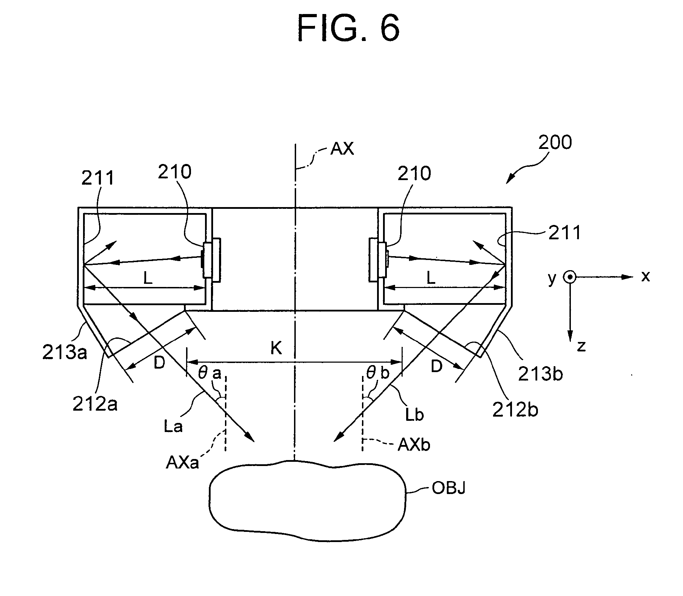

Next, a further detailed structure of the illuminating unit 200 will be described by referring to Fig. 5 and Fig. 6. Fig. 5 shows a structure of the illuminating unit 200 as viewed from the side of the object OBJ. The diffusing section 211 is structured to be the square cylindrical shaped. Moreover, the light source section 210 is disposed on four sides round the square cylindrical shape. The light source section 210 is formed by three sets of LEDs with each set including eight LEDs 210a, 210b, 210c, 210d, 210e, 210f, 210g, and 210h. In other words, the light source section 210 is made of total of 24 (= 3 sets x 8) LEDs. Each of the LEDs 210a to 210h allows the illuminating light to emerge toward the four sides around the diffusing section 221 having the square cylindrical shape.

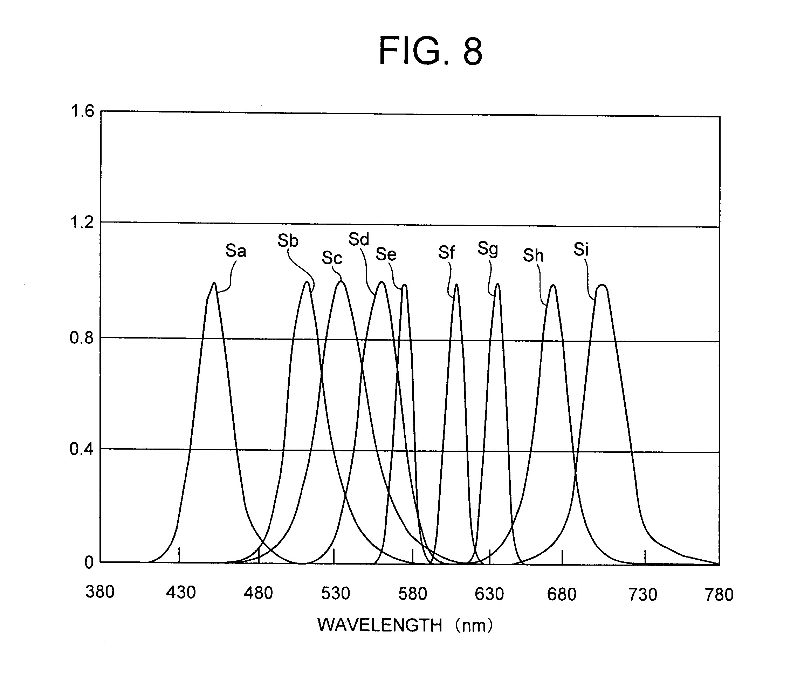

Fig. 8 shows a light emission spectrum of the eight LEDs 210a, 210b, 210c, 210d, 210e, 210f, 210g, and 210h. In Fig. 8, a horizontal axis indicates a wavelength (unit: nm) and a vertical axis indicates a light intensity (unit: arbitrary unit). The LED 210a has a spectral distribution with a central emission wavelength near 450 nm as shown in a curve Sa. The LED 210b has a spectral distribution with a central emission wavelength near 505 nm as shown in a curve Sb. The LED 210c has a spectral distribution with a central emission wavelength near 525 nm as shown in a curve Sc. The LED 210d has a spectral distribution with a central emission wavelength near 560 nm as shown in a curve Sd. The LED 210e has a spectral distribution with a central emission wavelength 575 nm as shown in a curve Se. The LED 210f has a spectral distribution with a central emission wavelength 609 nm as shown in a curve Sf. The LED 210g has a spectral distribution with a central emission wavelength 635 nm as shown in a curve Sg. The LED 210h has a spectral distribution with a central emission wavelength 670 nm as shown in a curve Sh. A curve Si will be used in a third embodiment which will be described later.

The description will be continued upon coming back to Fig. 5. As it has been described above, the LED controller 115 controls an emission state of the LEDs 210a to 210h. For example, the LED controller 115 puts ON the LED 210a which has the spectral distribution shown in the curve Sa, and puts OFF the other LEDs 210b to 210h. Further, the CCD 113 captures an image of image data by illuminating light of the spectral distribution shown in the curve Sa.

Next, the LED controller 115 puts OFF the LED 210a and puts on the LED 210b having the spectral distribution shown in the curve Sb. The CCD 113 captures an image of image data by illuminating light of the spectral distribution shown in the curve Sb. Further, the procedure of putting ON and putting OFF the light is performed repeatedly for all the LEDs 210a to 210b. The LEDs are suitable for speedy lighting up and lighting out control.

Light from the LEDs 210a to 210h is advanced toward the four sides around circumference of the square cylinder shaped diffusing section 211. Further, the light from the LEDs 210a to 210h receives a diffusion effect at an inner surface of the diffusing section 211. The light which is diffused is emerged either from the first aperture section 212a or from the second aperture section 212b. The first aperture section 212a and the second aperture section 212b form an aperture section. Each of the first aperture section 212a and the second aperture section 212b has an emerging section having a rectangular shape.

Fig. 6 shows a cross-sectional structure of the illuminating unit 200. The diffusing section 221 has a hollow cylindrical shape of a square pole. A detailed structure of the diffusing section 211 will be described later.

Here, axes La and Lb parallel to the axis AX of the imaging optical system 121 are taken into consideration. A light beam La emerged from the first aperture section 212a is advanced from left to right in Fig. 6. The light beam La makes and angle θa with the axis AXa, and illuminates the object OBJ. Similarly, a light beam Lb emerged from the second aperture section 212b is advanced from right to left in Fig. 6. The light beam Lb makes an angle θb with an axis AXb, and illuminates the object OBJ.

As it has been described above, the first aperture section 212a and the second aperture section 212b are provided facing mutually with respect to the axis AX of the imaging optical system 121. Therefore, it is possible to illuminate the object OBJ at the predetermined angles θa and θb from two mutually facing directions with the axis AX of the imaging optical system 121 as the center. Accordingly, the object OBJ is illuminated upon being superimposed by the light from the first aperture section 212a and the light from the second aperture section 212b. The angles θa and θb are let to be 45° or more than 45° for example.

Accordingly, incidence of specularly reflected light from the irradiated surface of the object OBJ on the imaging optical system 121 can be reduced. As a result of this, the imaging optical system 121 can fetch with high efficiency only the scattered light from the irradiated surface of the object OBJ. Consequently, it is possible to acquire the imaging apparatus in which the specularly reflected light is reduced, and which can measure with high accuracy the color distribution of the surface of the obj ect OBJ. Furthermore, the illuminating unit 200 illuminates the object OBJ from two different directions. Therefore, no shade is developed even for a three-dimensional object such as a tooth.

Moreover, the illuminating unit 200 is provided in an optical path between the imaging optical system 121 and the irradiated surface of the object OBJ. Further, it is more desirable to dispose the illuminating unit 200 near a side of incidence of the imaging optical system 121. Therefore, it is possible to illuminate from two directions using one illuminating unit 200. Furthermore, the imaging optical system 121 captures an image of the scattered light which has passed through the illuminating unit 200. Therefore, it is possible to simplify a structure around the imaging optical system. Consequently, it is possible to acquire a small sized imaging apparatus 100.

Moreover, a roof portion 213a is formed at an outer circumferential side of the first aperture section 212a. Furthermore, a roof portion 213b is formed at an outer circumferential side of the second aperture section 212b. The roof portions 213a and 213b are formed to have an angle of inclination and a size such that the diffused light emerged from the first aperture section 212a and the second aperture section 212b is guided efficiently toward the object OBJ.

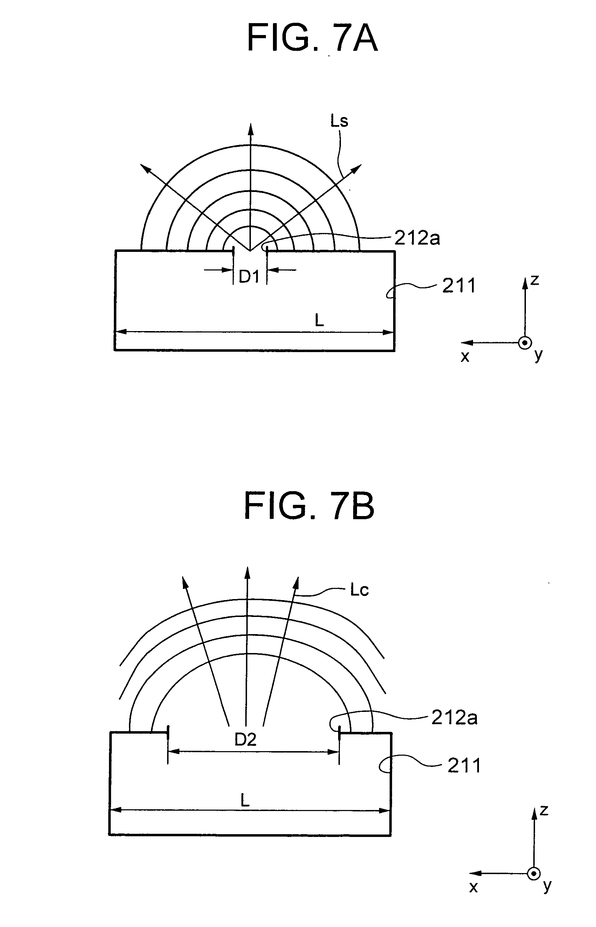

Fig. 7A and Fig. 7B are diagrams showing a relation between a size of the first aperture section 212a and an emerged light. Since the second aperture section 212b has the same structure as the first aperture section 212a, the description will be made with the first aperture section 212a as a representative example. As it has been described above, the diffusing plate 211 has the hollow cylindrical shape of a square pole. As shown in Fig. 7A, a cross-sectional shape of the diffusing section 211 along an x-y plane is a rectangular shape. A length of the rectangular shape in a direction along x axis is let to be L and an aperture diameter (length) of the first aperture section 212a is let to be D1. Fig. 7B shows a structure of the first aperture section 212a having an aperture diameter D2 which is greater compared the aperture diameter D1.

In Fig. 7A, when D1/L is sufficiently small, the first aperture section 212a functions as a point light source. Therefore, light Ls emerged from the first aperture section 212a becomes a substantially spherical wave. Whereas, as shown in Fig. 7B, when D2/L is a predetermined value, light Lc emerged from the first aperture section 212a becomes a substantially parallel light.

In the first embodiment, when the length of the diffusing section 211 along a predetermined plane (x-z plane in Fig. 6) is let to be L, and a length of the second aperture section 212b along a predetermined plane (x-z plane) is let to be D, the apertures is formed to have a ratio D/L so as to allow to emerge the substantially parallel light.

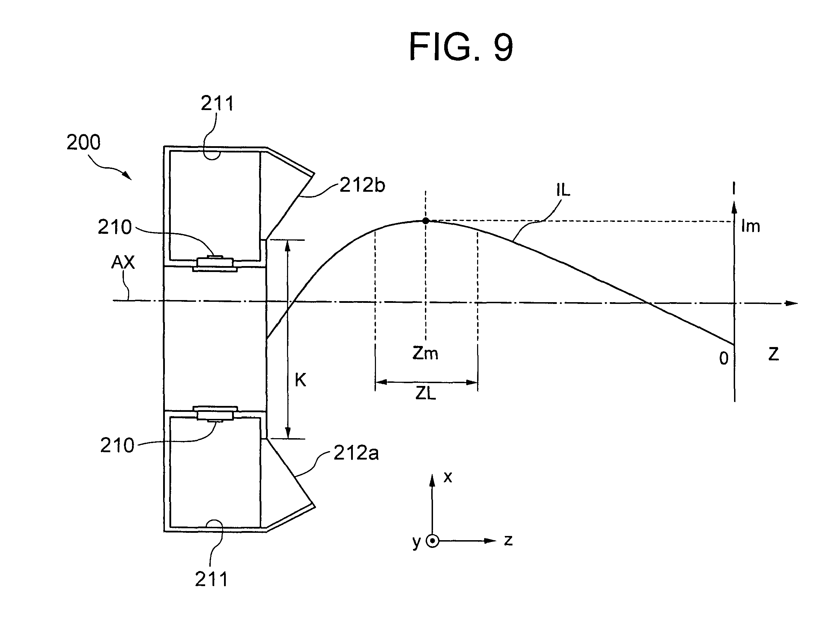

Fig. 9 shows both a structure of the illuminating unit 200 and a light intensity distribution IL of the illuminated light on the optical axis AX. An intensity distribution on the optical axis AX of the light from the first aperture section 212a and the second aperture section 212b is roughly zero near the aperture section. As going away from the aperture section, the light intensity distribution IL is increased and becomes the maximum value Im at a position Zm. Next, as going farther away from the position Zm, the light intensity distribution is gradually attenuated. Here, there is almost no change in the light intensity distribution in a direction along the optical axis AX in a range ZL, and is a substantially constant value. For example, even when an imaging distance between the imaging apparatus 100 and the object OBJ is changed in the range ZL, the light intensity distribution of the illuminated light is substantially constant. In other words, even when the imaging distance in the range ZL in a direction of focal depth, the imaging apparatus 100 can perform a satisfactory measurement with constant intensity distribution of the illuminated light.

Moreover, the first aperture section 212a and the second aperture section 212b are provided to be separated only by a predetermined interval K. By changing the interval K between the two aperture sections, it is possible to change the position Zm of the maximum value Im. For example, when the interval K is let to be small, the position Zm comes closer to the illuminating unit 200. Whereas, when the interval K is let to be large, the position Zm is moved in a direction going away from the illuminating unit 200. Furthermore, when the position Zm is moved away from the illuminating unit 200, an amount of the illuminating light is attenuated. Therefore, it is necessary to increase an output of the LEDs. Moreover, when the position Zm is too close to the illuminating unit 200, in a case of a three-dimensional object, sometimes the illuminating unit 200 and the object OBJ interfere. To give a concrete example, when a tooth is measured as an object OBJ, in the imaging apparatus having a small interval K between the two aperture sections, a nose of a human being and the imaging apparatus 100 make a contact. Thus, it is desirable to set the interval K to be an appropriate value according to the amount of light and a type of the object OBJ.

As it has been described in Fig. 7A, when the substantially spherical wave is emerged from the first aperture section 212a and the second aperture section 212b, if the position on the optical axis AX differs, a change in the light intensity distribution becomes large. Whereas, as shown in Fig. 7B, when the substantially parallel light is emerged from the first aperture section 212a and the second aperture section 212b, the change in the light intensity distribution according to the position along the optical axis AX is reduced as compared to the change in the light intensity distribution in Fig. 7A

Thus, in the first embodiment, the first aperture section 212a and the second aperture section 212b have the aperture diameter D2 of a size such that the illuminated light which is scattered at the diffusing section 211 is emerged as the substantially parallel light (refer to Fig. 7B). Accordingly, the light intensity distribution in the x-y plane in a direction substantially perpendicular to the optical axis AX is uniform, and the change in the amount of light in a z direction along the optical axis AX is reduced. Therefore, it is possible to measure with high accuracy the color distribution of the surface of the object OBJ. Light emerged through the first aperture section 212a and the second aperture section 212b is not restricted to the substantially parallel light, and may be gentle (having a large radius of curvature) convergent light or gentle divergent light.

### (Structure of diffusing section)

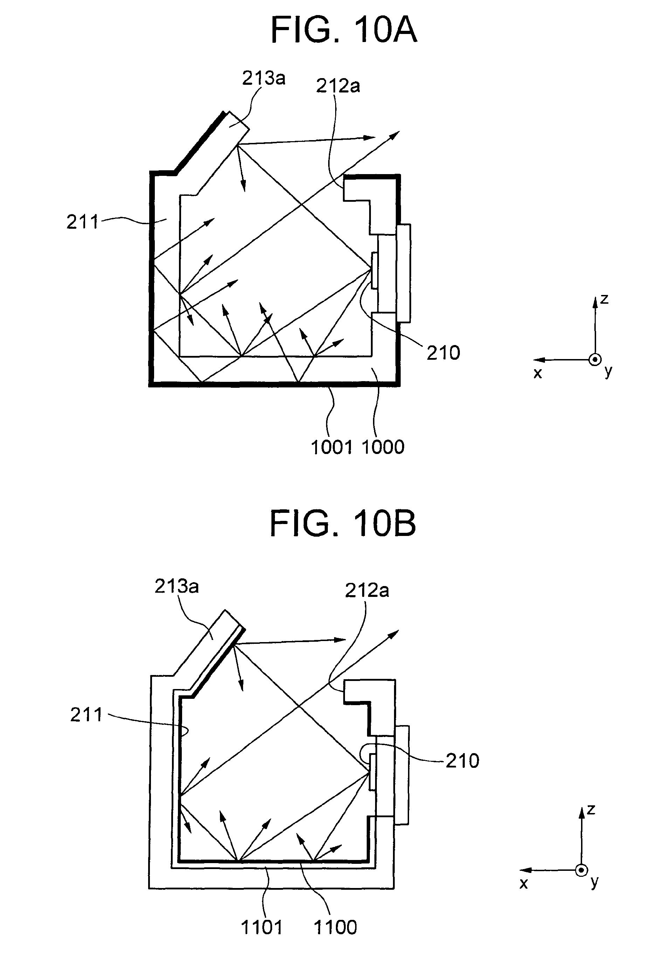

Fig. 10A shows a cross-sectional structure of the diffusing section 211. The diffusing section 211 includes a resin portion 1000. A thickness of the resin portion 1000 is about 1 mm to 2 mm for example. On a surface of the resin portion 1000, on a side opposite to the light source section 210, an aluminum layer 1001 is formed by metal deposition. The resin portion 1000 diffuses light from the light source section 210. Moreover, the aluminum layer 1001 reflects light which is passed through the thin resin portion 1000. The reflected light is scattered by being incident again on the resin portion 1000. Accordingly, it is possible to reduce the amount of light which is lost by transmission through the resin portion 1000.

As it has been mentioned above, the light source section 210 is formed by the LEDs 210a to 210h, each supplying light of different wavelength range. Further, a position at which each of the LEDs 210a to 210h is installed is different. Therefore, for example, for light emitted from the LED 210a and light emitted from the LED 210h, optical characteristics such as an illuminance distribution differ. The resin portion 1000 scatters light from each of the LEDs 210a to 210h. Accordingly, it is possible to reduce a variation in the optical characteristics caused due to a difference in disposing the LEDs 210a to 210h.

Moreover, the roof portion 213 is formed near the first aperture section 212a. The roof portion 213a, as it has been described above, has the angle of inclination and the size such that the scattered light is guided efficiently toward the object OBJ. For example, when the roof portion 213a is not provided, light not advancing toward the object OBJ is generated. Therefore, the amount of light is lost. Moreover, when the roof portion 213a is provided perpendicularly (parallel to the optical axis AX), the light which is specularly reflected at the roof portion 213a is incident on the object OBJ. Thus, the roof portion 213a also serves a function of returning the light specularly reflected at the roof section 213a, out of the light from the light source section 210, toward an inside of the diffusing section 211. Therefore, it is possible to reduce incidence of the specularly reflected light from the roof portion 213a, on the object OBJ.

Fig. 10B shows a cross-sectional structure of a modified example of the diffusing plate 211. A reflecting coat layer 1101 is formed on an inner surface of the diffusing section 211. A diffusing coat layer 1100 is further formed on the reflecting coat layer 1101. The diffusing coat layer 1100 can be formed by applying barium sulfate for example. Accordingly, the diffusing coat layer 100 diffuses the light which is incident. Thus, a film of a high reflectance (coat layer) and a film of a high diffusivity (coat layer) may be stacked in two layers, or may be formed by using other material. Moreover, in the first embodiment, the light source sections 210 are disposed at four sides of the illuminating unit 200. However, it is not required to necessarily dispose the light source sections 210 at all four sides. The structure may such that in an acceptable range in which there does not arise a problem of an effect of a change of color and color distribution including a change in a brightness and light distributing characteristics of a light source, the light source sections 210 are disposed on two sides facing one another in a horizontal direction or in a vertical direction and the light source sections 210 are not disposed on the remaining two side, or the light source sections 210 are disposed on three sides and the light source section 210 is not disposed on one side. In this case, effects such as a decrease in the number of LEDs used and a reduction in the size of the light source section can be desired.

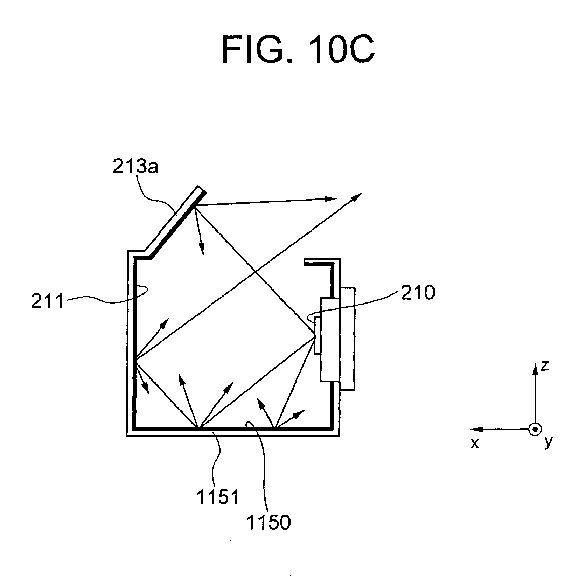

Fig. 10C shows another modified example of the diffusing section 211. For example, a diffusing coat layer 1150 is formed on an aluminum substrate 1151 which is a reflecting surface. Accordingly, it is possible to manufacture the diffusing section 211 easily by using the aluminum substrate 1151.

In any of the structures in Fig. 10A to Fig. 10C, it is possible to make uniform the intensity distribution of the illuminated light by improving the diffusion characteristics of the diffusing section 211. Moreover, by improving the diffusion characteristics, it is possible to improve an efficiency of light. Furthermore, each of the LED 210a to 210h is disposed to emit the light toward the outer circumference of the diffusing section 211. Accordingly, it is possible to reduce direct emergence of light supplied from each of the LEDs 210a to 210h, from the first aperture section 212a and the second aperture section 212b. According to this arrangement, it is possible to improve a light diffusing efficiency of the diffusing section 211.

A lens may be disposed near the first aperture section 212a and the second aperture section 212b. The lens has a function of guiding toward the object OBJ, upon allowing to refract the light diffused at the diffusing section 211. Accordingly, it is possible to illuminate the object efficiently by the light which is diffused.

### (Second embodiment)

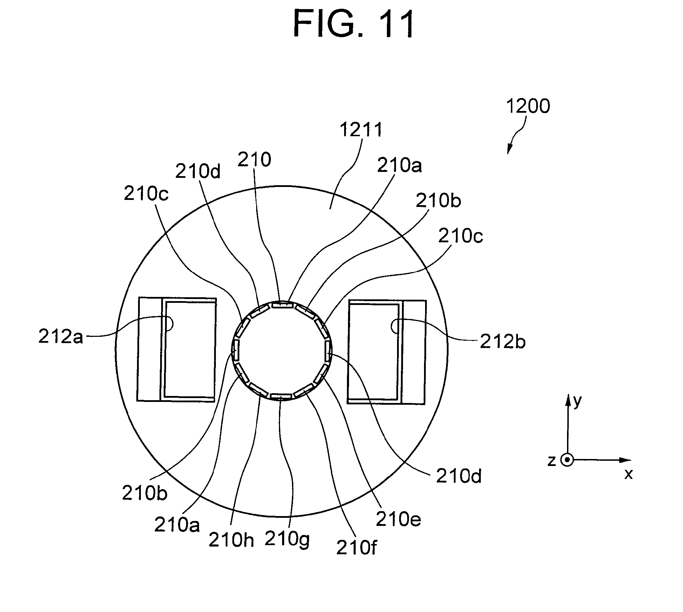

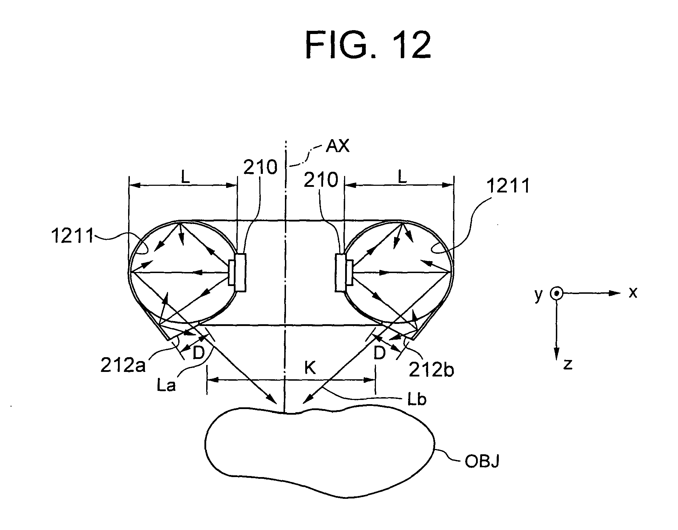

A structure of an illuminating unit 1200 according to a second embodiment will be described by referring to Fig. 11 and Fig. 12. Same reference numerals are assigned to components same as in the first embodiment, and the description to be repeated is omitted. Fig. 11 shows a structure of the illuminating unit 1200 as seen from the side of the object OBJ. Moreover, Fig. 12 shows a cross-sectional structure of the illuminating unit 1200. As shown in Fig. 11, the diffusing section 211 is formed to have an annular cylindrical shape. The light source section 210 is disposed on an inner circumferential side of the annular cylindrical shape. The light source section 210 includes the LEDs 210a, 210b, 210c, 210d, 210e, 210f, 210g, and 210h. In Fig. 11, two LEDs each of the LED 210a, 210b, and 210c are shown. Thus, the illuminating unit 1200 has total of 12 LEDs disposed at substantially equal interval. Each of the LEDs 210a to 210h is structured to emit light toward the outer circumference of the diffusing section 211.

As shown in Fig. 12, a cross section of a diffusing section 1211 is circular shaped. Due to this shape, it is possible to improve the efficiency of the illuminating light. Moreover, similarly as in the first embodiment mentioned above, when the length of the diffusing section 1211 along the predetermined plane (x-z plane in Fig. 12) is let to be L, and a length of the first aperture section 212a and the second aperture section 212b along a predetermined plane is let to be D, the aperture is formed to have a ratio D/L so as to allow to emerge the substantially parallel light.

Moreover, the first aperture section 212a and the second aperture section 212b are provided to be separated only by a predetermined distance K such that the position Zm (refer to Fig. 9) of the maximum value Im is optimum.

In the second embodiment, 12 LEDs 802a to 802h are repeatedly put ON and OFF one after the other. Further, the object OBJ is illuminated by the illuminated light from the LED which is put ON. Moreover, a position at which each of the LEDs 802a to 802h is disposed in a circular shape is different. The diffusing plate 1211 generates diffused light having a substantially uniform intensity distribution, irrespective of the light distribution characteristics of each of the LEDs 802a to 802h. Therefore, it is possible to reduce a variation in the optical characteristics caused due to a difference in the positions of the LEDs. Moreover, the diffusing section 1211 is not restricted to be annular cylindrical shaped as shown in Fig. 11. For example, the diffusing section 1211 may let to be elliptical annular cylindrical shaped having a major axis and a minor axis, when viewed from the side of the object OBJ. At this time, it is desirable that the first aperture section 212a and the second aperture section 212b are provided in a direction along the major axis. Accordingly, it is possible to reduce the size while improving the diffusion efficiency.

### (Third embodiment)

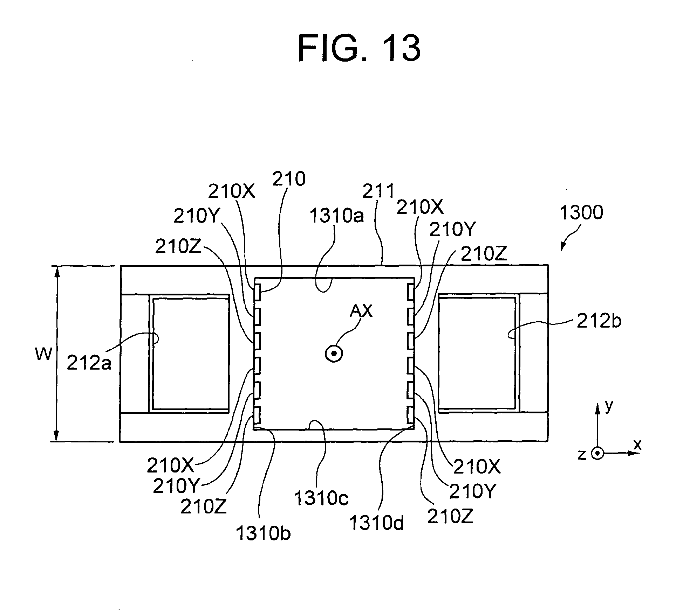

A structure of an illuminating unit 1300 according to a third embodiment will be described below by referring to Fig. 13. Same reference numerals are assigned to components same as in the first and the second embodiment, and the description to be repeated is omitted. Fig. 13 shows a structure of the illuminating unit 1300 as viewed from the side of the object OBJ.

The diffusing section 211 is formed to have a continuous cylindrical shape encircling the optical axis AX which is a predetermined axis. Moreover, by the cylindrical shaped diffusing section 211, a space of a predetermined shape is formed near the optical axis AX which is the predetermined axis. Light from the illuminating unit 1300 is reflected at the surface of the object OBJ, and scattered. The scattered light is passed through the space having the predetermined shape, of the illuminating unit 1300, and is incident on the imaging optical system 121. Thus, it is possible to dispose the illuminating unit 1300 near an end surface of incidence of the imaging optical system 121. As a result, it is possible to reduce a size of the entire imaging apparatus.

Moreover, the space having the predetermined shape is formed to be substantially square shaped by four surfaces (sides) 1310a, 1310b, 1310c, and 1310d on an inner circumference of the diffusing section 211. An imaging surface of the CCD 113 has a square shape. Therefore, when the size of the imaging apparatus is reduced, it is possible to reduce a vignetting of light due to a peripheral portion of the square shaped space of the illuminating unit 1300.

Moreover, the light source section 210 is formed by four sets of LEDs, each set including three LEDS 210X, 210Y, and 210Z. In other words, the light source section 210 is made of total of 12 (= 4 sets x 3) LEDs. Each of the LEDs 210X, 210Y, and 210Z is provided on the two surfaces 1310b and 1310d of the diffusing section 211 on a side of the optical axis AX. Similarly as in the first embodiment and the second embodiment, the first aperture section 212a and the second aperture section 212b are provided at the positions facing each other with respect to the optical axis AX. Further, the light source section 210 is provided on the surface of the diffusing section 211 on the side of the optical axis AX or near the surface of the diffusing section 211 on the side of the optical axis AX. Concretely, each of the LEDs 210X, 210Y, and 210Z is provided on the surfaces 1310b and 1310d on the inner side of the diffusing section 211. Moreover, each of the LEDs 210X, 210Y, and 210Z emits illuminating light from the inner circumference of the cylinder shaped diffusing section 211 toward four sides on the outer circumference. As a result, it is possible to structure the diffusing section 211 compactly. Moreover, it is possible to make a width W of the diffusing section small. Accordingly, it is possible to reduce the size of the illuminating unit 1300 and to save the space. Furthermore, by disposing the light source section 210 on the inner side, it is possible to improve the diffusion efficiency of light in the diffusing section 211. Consequently, it is possible to illuminate the object OBJ by bright illuminating light. As compared to the annular cylinder shaped diffusing section in the second embodiment, by structuring the diffusing section to have a square cylindrical shape as in the third embodiment, it is possible to further increase the diffusion efficiency.

Moreover, the LEDs 210X, 210Y, and 210Z are provided at positions such that the illuminating light supplied from the LEDs 210X, 210Y, and 210Z is emerged from the aperture sections 212a and 212b upon reflecting at least once at the diffusing section 211. In other words, when the aperture sections 212a and 212b are observed from the side of the object OBJ, the LEDs 210X, 210Y, and 210Z are disposed at positions at which, direct light from the LEDs 210X, 210Y, and 210Z, in other words light which is not reflected even once at the diffusing section 211, cannot be observed. It is desirable that the aperture sections 212a and 212b allow the diffused light to be emerged efficiently. In the third embodiment, the light supplied from the LEDs 210X, 210Y, and 210Z is emerged from the aperture sections 212a and 212b upon being reflected once without fail at the diffusing section 211. Therefore, from among the light emitted from the LEDs 210X, 210Y, and 210Z, it is possible to reduce light which is emerged directly from the aperture sections 212a and 212b without being reflected at the diffusing section 211. As a result of this, the illuminating unit 1300 can illuminate the object OBJ by satisfactory diffused light.

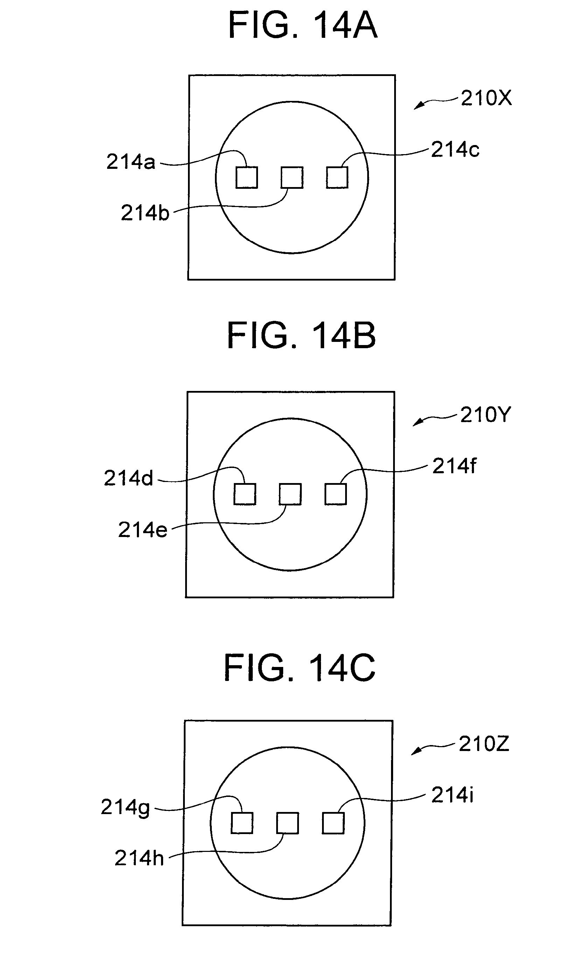

Next, a structure of each of the LEDs 210X, 210Y, 210Z will be described. Fig. 14A, Fig. 14B, and Fig. 14C show a structure of each of the LEDs 210X, 210Y, and 210Z as viewed from a front side. As shown in Fig. 14A, the LED 210X has three light emitting sections 214a, 214b, and 214c. Moreover, as shown in Fig. 14B, the LED 210Y has three light emitting sections 214d, 214e, and 214f. Furthermore, as shown in Fig. 14C, the LED 210Z has three light emitting sections 214g, 214h, and 214i.

A spectral distribution of the illuminating light which is supplied from each of the emitting sections will be described by referring to Fig. 8. The light emitting section 214a has a spectral distribution with a central emission wavelength near 450 nm as shown in the curve Sa. The light emitting section 214b has a spectral distribution with a central emission wavelength near 505 as shown in the curve Sb. The light emitting section 214c has a spectral distribution with a central emission wavelength near 525 nm as shown in curve Sc. Thus, the LED 210X can supply light of three different wavelength ranges from one package.

Moreover, the light emitting section 214d has a spectral distribution with a central emission wavelength near 560 nm as shown in the curve Sd. The light emitting section 214e has a spectral distribution with a central emission wavelength near 575 nm as shown in the curve Se. The light emitting section 214f has a spectral distribution with a central emission wavelength 609 nm as shown in the curve Sf. Thus, the LED 210Y can supply light of three different wavelength ranges from one package.

Furthermore, the light emitting section 214g has a spectral distribution with a central emission wavelength 635 nm as shown in the curve Sg. The LED 214h (light emitting section 214h) has a spectral distribution with a central emission wavelength 670 nm as shown in the curve Sh. The LED 214i (light emitting section 214i) has a spectral distribution with a central emission wavelength 700 nm as shown in the curve Si. Thus, the LED 210Z can supply light of three different wavelength ranges from one package.

Accordingly, from the three LEDs 210X, 210Y, and 210Z, the light of nine different wavelength ranges, in other words nine bands, can be supplied. As a result of this, it is possible to reduce a variation in a light emitting position of light for each different wavelength range. Consequently, it is possible to reduce a change in illumination unevenness according to illuminating light of different colors. The emission wavelength range of each of the nine light emitting sections 214a to 214i may not be let to be different. For example, from among the nine light emitting sections 214a to 214i, the light emission wavelength of the two light emitting sections may be let to be substantially same. At this time, light of eight different wavelength ranges, in other words light of eight bands can be supplied by the nine light emitting sections 214a to 214i.

Similarly, it is possible to control the number of bands of the light supplied by the LEDs 210X, 210Y, and 210Z to appropriate desirable number. For example, it is possible to structure each of the LEDs 210X, 210Y, and 210Z to have three light emitting sections, each supplying R light (red color), G light (green color), and B light (blue color). According to this structure, it is possible to acquire a three-band color camera of R light, G light, and B light.

Moreover, it is possible to use an LED which supplies white color light, as the light source section. In this case, a color CCD which includes an R light transmission filter, a G light transmission filter, and a B light transmission filter is used as the CCD 113. Moreover, the object OBJ is illuminated by the white color light. Light scattered from the object OBJ is imaged at the color CCD. When three types of color filters are used, it is equivalent to a three-band illumination. Consequently, when the number of color filters of different transmission wavelength range is increased, it is possible to have multiple bands according to the number of color filters.

The shape of the diffusing section, the mode of disposing the LEDs, and the size and the position of the aperture section are not restricted to the embodiments described above. For example, when the diffusing section without being restricted to the continuous cylindrical structure, has a space for diffusing, any structure may be used.

Furthermore, the LED having a plurality of light emitting sections in one package as shown in the third embodiment may be used in the illuminating unit of the first embodiment or the second embodiment. Thus, the present invention is not restricted to each of the embodiments mentioned above, and various modifications in a scope which fall within the basic teaching of the present invention are possible

### INDUSTRIAL APPLICABILITY

Thus, it is possible to use appropriately an illuminating unit according to the present invention at the time of illuminating in an imaging apparatus which measures a color distribution of a surface of an object.

## Claims

1. An illuminating unit comprising:
a light source section which supplies an illuminating light;
a diffusing section which diffuses by reflecting the illuminating light from the light source section; and
an aperture section which allows a diffused illuminating light to emerge, wherein
the aperture section has a diameter which allows the diffused illuminating light to emerge as a substantially parallel light.

2. An illuminating unit comprising:
a light source section which supplies an illuminating light;
a diffusing section which diffuses by reflecting the illuminating light from the light source section; and
an aperture section which allows a diffused illuminating light to emerge, wherein
the light source section has a plurality of light emitting sections, each supplying light of different wavelength range, and
the aperture section has a diameter which allows the diffused illuminating light to emerge as a substantially parallel light.

3. An illuminating unit comprising:
a light source section which supplies an illuminating light;
a diffusing section which diffuses by reflecting the illuminating light from the light source section; and
an aperture section which allows a diffused illuminating light to emerge, wherein
the light source section is provided at a position at which the illuminating light supplied by the light source section is emerged from the aperture section upon being reflected at least once in the diffusing section.

4. An illuminating unit comprising:
a light source section which supplies an illuminating light;
a diffusing section which diffuses by reflecting the illuminating light from the light source section; and
an aperture section which allows a diffused illuminating light to emerge, wherein
the light source section has a plurality of light emitting sections, each supplying light of different wavelength range, and
the light source section is provided at a position at which the illuminating light supplied by the light source section is emerged from the aperture section upon being reflected at least once in the diffusing section.

5. The illuminating unit according to any one of claims 1 to 4, wherein
the aperture section includes a first aperture section and a second aperture section which are provided at a predetermined interval.

6. The illuminating unit according to claim 5, wherein
the first aperture section and the second aperture section are provided at positions facing with respect to a predetermined axis.

7. The illuminating unit according to claim 6, wherein
the light source section is provided on a surface of the diffusing plate, on a side of the predetermined axis, or near the surface of the diffusion plate, on the side of the predetermined axis.

8. The illuminating unit according to claim 6, wherein
the diffusing section is formed to be continuous cylindrical shaped, encircling the predetermined axis, and
a space of a predetermined shape is formed near the predetermined axis, by the cylindrical shaped diffusing section.

9. The illuminating unit according to claim 8, wherein
the space of the predetermined shape is substantially square shaped.

10. The illuminating unit according to one of claims 1 and 2, wherein
the light source section is provided at a position at which the illuminating light supplied by the light source section is emerged from the aperture section upon being reflected at least once in the diffusing section.

11. An imaging apparatus comprising:
the illuminating unit according to any one of claims 1 to 10; and
an imaging optical system which forms an image of a reflected light from an irradiated surface which is illuminated by the illuminating unit, wherein
a color distribution of the irradiated surface is calculated based on the reflected light acquired by the imaging optical system.

## Amended claims

### Amended claims under Art. 19.1 PCT

1. (amended) An illuminating unit which is used in an imaging apparatus which includes an imaging optical system, comprising:
a light source section which supplies an illuminating light;
a diffusing section which diffuses by reflecting the illuminating light from the light source section; and
an aperture section which allows a diffused illuminating light to emerge, wherein
the aperture section has a diameter which allows the diffused illuminating light to emerge as a substantially parallel light, and
an angle between an optical axis of the imaging optical system and the substantially parallel light is 45° or greater than 45°.

2. (amended) An illuminating unit which is used in an imaging apparatus which includes an imaging optical system, comprising:
a light source section which supplies an illuminating light;
a diffusing section which diffuses by reflecting the illuminating light from the light source section; and
an aperture section which allows a diffused illuminating light to emerge, wherein
the light source section has a plurality of light emitting sections which supply light of different wavelength range respectively, and
the aperture section has a diameter which allows the diffused illuminating light to emerge as a substantially parallel light, and
an angle between an optical axis of the imaging optical system and the substantial parallel light is 45° or greater than 45°.

3. (amended) An illuminating unit which is used in an imaging apparatus which includes an imaging optical system, comprising:
a light source section which supplies an illuminating light;
a diffusing section which diffuses by reflecting the illuminating light from the light source section;
a diffusing section which diffuses by reflecting the illuminating light from the light source section; and
an aperture section which allows a diffused illuminating light to emerge, wherein
the light source section is provided at a position at which the illuminating light supplied by the light source section is emerged from the aperture section, upon being reflected at least once in the diffusing section, and
an angle between an optical axis of the imaging optical system and the substantially parallel light is 45° or greater than 45°.

4. (amended) An illuminating unit which is used in an imaging apparatus which includes an imaging optical system, comprising:
a light source section which supplies an illuminating light;
a diffusing section which diffuses by reflecting the illuminating light from the light source section; and
an aperture section which allows a diffused illuminating light to emerge, wherein
the light source section has a plurality of light emitting sections, each supplying light of different wavelength range, and
the light source section is provided at a position at which the illuminating light supplied by the light source section is emerged from the aperture section upon being reflected at least once in the diffusing section, and
an angle between an optical axis of the imaging optical system and the substantially parallel light is 45° or greater than 45°.

5. The illuminating unit according to any one of claims 1 to 4, wherein
the aperture section includes a first aperture section and a second aperture section which are provided at a predetermined interval.

6. The illuminating unit according to claim 5, wherein
the first aperture section and the second aperture section are provided at positions facing with respect to a predetermined axis.

7. The illuminating unit according to claim 6, wherein
the light source section is provided on a surface of the diffusing plate, on a side of the predetermined axis, or near the surface of the diffusion plate, on the side of the predetermined axis.

8. The illuminating unit according to claim 6, wherein
the diffusing section is formed to be continuous cylindrical shaped, encircling the predetermined axis, and
a space of a predetermined shape is formed near the predetermined axis, by the cylindrical shaped diffusing section.

9. The illuminating unit according to claim 8, wherein
the space of the predetermined shape is a substantially square shaped.

10. The illuminating unit according one of claims 1 and 2, wherein
the light source section is provided at a position at which the illuminating light supplied by the light source section is emerged from the aperture section upon being reflected at least once in the diffusing section.

11. (amended) An imaging apparatus comprising:
the illuminating unit according to any one of claims 1 to 10; and
the imaging optical system which forms an image of a reflected light from an irradiated surface which is illuminated by the illuminating unit, wherein
a color distribution of the irradiated surface is calculated based on the reflected light acquired by the imaging optical system.
